(19) Europäisches Patentamt
European Patent Office
Office européen des brevets

(11) Veröffentlichungsnummer: **0 089 585**
**B1**

(12) **EUROPÄISCHE PATENTSCHRIFT**

(45) Veröffentlichungstag der Patentschrift: **19.12.84**

(51) Int. Cl.³: **C 07 C 69/16**, C 07 C 67/293

(21) Anmeldenummer: **83102451.8**

(22) Anmeldetag: **12.03.83**

# (54) Verfahren zur Herstellung von 2-Alkyl-4,4-diacyloxy-2-butenalen.

(30) Priorität: **24.03.82 DE 3210706**

(43) Veröffentlichungstag der Anmeldung: **28.09.83 Patentblatt 83/39**

(45) Bekanntmachung des Hinweises auf die Patenterteilung: **19.12.84 Patentblatt 84/51**

(84) Benannte Vertragsstaaten: **CH DE FR GB LI**

(56) Entgegenhaltungen:
**EP-A- 0 005 452**
**EP-A- 0 068 372**

(73) Patentinhaber: **BASF Aktiengesellschaft
Carl-Bosch-Strasse 38
D-6700 Ludwigshafen (DE)**

(72) Erfinder: **Fischer, Rolf Hartmuth, Dr.
Bergstrasse 98
D-6900 Heidelberg (DE)**
Erfinder: **Weitz, Hans Martin, Dr.
Auf dem Koeppel 40
D-6702 Bad Duerkheim (DE)**
Erfinder: **Paust, Joachim, Dr.
Ringstrasse 3
D-6701 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 089 585 B1

**Beschreibung**

Die Erfindung betrifft ein Verfahren zur Herstellung von 2-Alkyl-4,4-diacyloxy-2-butenalen durch Isomerisierung von 2-Alkyl-2,4-diacyloxy-3-butenalen in Gegenwart von aliphatischen Carbonsäuren.

Es ist bekannt, daß sich in 4-Stellung substituierte 3-Alkyl-3-acetoxy-1-butene in Gegenwart von Saüren zu den entsprechenden 1-Acetoxy-3-alkyl-2-butenen isomerisieren lassen (« Allylumlagerung »). So geht z. B. 3-Methyl-3-acetoxy-4-chlor-1-buten in Gegenwart von Schwefelsäure, Essigsäure und Kupfersulfat in 1-Acetoxy-3-methyl-4-chlor-2-buten über (Journ. Amer. Chem. Soc. *72,* 4608 (1950)). 3-Methyl-3-acetoxy-4-nitro-1-buten läßt sich in Gegenwart von konzentrierter Schwefelsäure in 1-Acetoxy-3-methyl-4-nitro-2-buten umwandeln (J. Org. Chem. *42,* 2939 (1977)). 3-Alkyl-3-hydroxy-1-butene, die in 4-Stellung z. B. durch Chlor (J. Org. Chem. *44,* 1716 (1979)), Phenyl-, Vinyl- oder n-Pentylreste (Tetrahedron Letters 351 (1974)) substituiert sind, lassen sich mit Essigsäure, Acetanhydrid und p-Toluolsulfonsäure in die entsprechenden, in 4-Stellung substituierten 1-Acetoxy-3-alkyl-2-butene überführen.

Es bestand nun die Aufgabe, 2-Alkyl-2,4-diacyloxy-3-butenale in 2-Alkyl-4,4-diacyloxy-2-butenale umzuwandeln.

Es wurde gefunden, daß man 2-Alkyl-4,4-diacyloxy-2-butenale der Formel

$$\begin{matrix} R^2-\overset{O}{\overset{\|}{C}}-O \\ R^3-\underset{\underset{O}{\|}}{C}-O \end{matrix}\!\!\!> CH-CH=\overset{R^1}{\overset{|}{C}}-C\!\!\begin{matrix}\nearrow O \\ \searrow H\end{matrix} \qquad (I)$$

in der $R^1$ einen Alkylrest mit 1 bis 5 Kohlenstoffatomen und $R^2$ und $R^3$ ein Wasserstoffatom, einen aliphatischen Rest mit 1 bis 15 Kohlenstoffatomen, einen cycloaliphatischen Rest mit 5 bis 7 Kohlenstoffatomen oder einen aromatischen Rest bedeutet, dadurch herstellen kann, daß man 2-Alkyl-2,4-diacyloxy-3-butenale der Formel

$$R^2-\overset{O}{\overset{\|}{C}}-O-CH=CH-\overset{R^1}{\overset{|}{\underset{\underset{\underset{\underset{R^3}{|}}{O=C}}{|}}{\underset{O}{|}}{C}}}-C\!\!\begin{matrix}\nearrow O \\ \searrow H\end{matrix} \qquad (II)$$

in der die Reste $R^1$ bis $R^3$ die obengenannte Bedeutung haben, in Abwesenheit von starken Säuren mit aliphatischen Carbonsäuren behandelt.

Nach dem neuen Verfahren werden die 2-Alkyl-4,4-diacyloxy-2-butenale aus den 2-Alkyl-2,4-diacyloxy-3-butenalen in einem technisch sehr einfachen Reaktionsschritt überwiegend als Trans-Verbindung erhalten.

Die Reaktion läßt sich für den Fall der Herstellung von 2-Methyl-4,4-diacetoxy-2-butenal formal durch die folgenden Formeln veranschaulichen :

$$AcO-CH=CH-\overset{CH_3}{\overset{|}{\underset{\underset{OAc}{|}}{C}}}-C\!\!\begin{matrix}\nearrow O \\ \searrow H\end{matrix} \xrightarrow{CH_3-COOH} \begin{matrix} AcO \\ AcO \end{matrix}\!\!> CH-CH=\overset{CH_3}{\overset{|}{C}}-C\!\!\begin{matrix}\nearrow O \\ \searrow H\end{matrix}$$

$$(OAc = O-\overset{O}{\overset{\|}{C}}-CH_3)$$

Die als Ausgangsstoffe verwendeten 2-Alkyl-2,4-diacyloxy-3-butenale der Formel II lassen sich durch Umsetzung von 2-Alkyl-1,4-diacyloxy-1,3-butadienen mit Sauerstoff abgebenden Verbindungen herstellen.

Die Ausgangsstoffe der Formel II enthalten als Alkylreste mit 1 bis 5 Kohlenstoffatomen z. B. Methyl-, Ethyl-, Propyl- oder Butylreste. Aliphatische Reste mit 1 bis 15 Kohlenstoffatomen sind z. B. Alkylreste, wie Methyl-, Ethyl-, n-Propyl-, i-Propyl-, n-Butyl-, i-Butyl-, Palmityl- oder Stearylreste. Als cycloaliphatische Reste kommen z. B. Cyclopentyl-, Cyclohexyl- oder Cycloheptylreste und als aromatische Reste

Phenylreste in Betracht. Beispielsweise seien die folgenden Ausgangsstoffe genannt : 2-Methyl-, 2-Ethyl-, 2 n-Propyl-, 2-Butyl-, 2-Pentyl-2,4-diacetoxy-3-buten, 2-Methyl-2-acetoxy-4-palmityloxy-3-buten und 2-Methyl-2-acetoxy-4-cyclohexyloxy-3-buten.

Das erfindungsgemäße Verfahren führt man in Abwesenheit von starken säuren, wie Mineralsäuren oder Sulfonsäuren durch, da die Ausgangsstoffe unter der Einwirkung starker Säuren überwiegend in 3-Alkyl-2,5-dihydrofuran-2-one übergehen.

Als aliphatische Carbonsäuren kommen z. B. Ameisensäure, Essigsäure, Propionsäure, Buttersäure, Valeriansäure, Palmitinsäure oder Stearinsäure in Betracht. Die Carbonsäuren können auch Wasser enthalten. Es ist auch möglich, in Anwesenheit eines unter den Reaktionsbedingungen inerten Lösungsmittels zu arbeiten. Lösungsmittel dieser Art sind z. B. Carbonsäureester, wie Essigsäuremethylester, chlorierte Kohlenwasserstoffe, wie Methylenchlorid, Chloroform, Tetrachlorkohlenstoff oder 1,2-Dichlorethan, Kohlenwasserstoffe, wie Alkane, Benzol und alkylierte Benzole oder Ether, wie Diethylether, Tetrahydrofuran und Dioxan.

Der Wassergehalt der jeweiligen Carbonsäure kann bis zu 20 Mol, insbesondere bis zu 5 Mol pro Mol eingesetztes 2-Alkyl-2,4-diacyloxy-3-butenal betragen.

Man behandelt die Ausgangsstoffe der Formel II mit den Carbonsäuren z. B. bei Temperaturen von 20 bis 200 °C, insbesondere bei 50 bis 120 °C. Die Behandlungsdauer beträgt etwa 0,5 bis 40 Stunden. Die Umlagerung kann drucklos oder unter Druck, diskontinuierlich oder kontinuierlich durchgeführt werden. die Konzentration der umzulagernden verbindungen in dem Behandlungsgemisch beträgt zweckmäßigerweise 1 bis 20 Gew.%.

Um möglichst hohe Ausbeuten an 2-Alkyl-4,4-diacyloxy-2-butenalen der Formel I zu erzielen, empfiehlt es sich, die Umlagerungstemperatur, die Umlagerungszeit, die Konzentration an der umzulagernden Verbindung und gegebenenfalls an Wasser in der jeweiligen carbonsäure aufeinander abzustimmen. So wendet man beispielsweise für hohe umlagerungstemperaturen kurze Umlagerungszeiten an.

Das erfindungsgemäße Verfahren wird z. B. wie folgt durchgeführt. Man löst das 2-Alkyl-2,4-diacyloxy-3-butenal in der jeweiligen Carbonsäure und erhitzt die Lösung auf die Umlagerungstemperatur. Nach beendeter Reaktion wird die Carbonsäure abdestilliert. Der Rückstand wird durch Destillation oder Kristallisation gereinigt.

Es war überraschend, daß bei der Behandlung der 2-Alkyl-2,4-diacyloxy-3-butenale mit den Carbonsäuren die gewünschten 2-Alkyl-4,4-diacyloxy-2-butenale entstehen, da bei Einwirkung von starken Säuren, wie Mineralsäuren oder Sulfonsäuren, 3-Alkyl-2,5-dihydrofuran-2-one gebildet werden.

Vorteilhaft ist, daß sich für die Herstellung der Verbindungen der Formel I nach dem erfindungsgemäßen Verfahren auch Gemische aus 2-Alkyl-2,4-diacyloxy-3-butenalen der Formel II und 2-Alkyl-4,4-diacyloxy-2-butenalen der Formel I, die sich destillativ nur äußerst schwierig trennen lassen, verwenden lassen. Vorteilhaft ist weiterhin, daß die verwendeten Carbonsäuren und das Wasser bei der Aufarbeitung destillativ, also ohne vorhergehende Neutralisation, abgetrennt werden können.

Die als Verfahrensprodukte erhältlichen 2-Alkyl-4,4-diacyloxy-2-butenale sind wertvolle Zwischenprodukte, z. B. für die Herstellung von Terpenen (DE-OS 23 57 810).

## Beispiel 1

Ein Gemisch aus 2,9 g 2-Methyl-2,4-diacetoxy-3-butenal und 1,5 g 2-Methyl-4,4-diacetoxy-2-butenal wurde in 50 g Eisessig gelöst und 4 Stunden auf 100 °C erhitzt. Nach dem Abziehen der Essigsäure am Rotationsverdampfer erhielt man durch Kugelrohrdestillation (120 °C/0,8 mbar) 3,0 g 2-Methyl-4,4-diacetoxy-2-butenal (70 %, bezogen auf eingesetzte Methyldiacetoxybutenale), dessen Struktur durch das $^1$H-NMR-Spektrum bestätigt wurde.

## Beispiel 2

Ein Gemisch aus 20,4 g 2-Methyl-2,4-diacetoxy-3-butenal und 14 g 2-Methyl-4,4-diacetoxy-2-butenal wurde mit 2,62 Gew.% Wasser enthaltender Essigsäure 2 Stunden auf 100 °C erhitzt. Nach dem Abziehen von Essigsäure und Wasser am Rotationsverdampfer erhielt man durch fraktionierte Destillation 17 g 2-Methyl-4,4-diacetoxy-2-butenal vom Siedepunkt 85 bis 93 °C/0,4 mbar (49 %, bezogen auf eingesetzte Methyldiacetoxybutenale) und 4,5 g 3-Methyl-2,5-dihydrofuran-2-on vom Siedepunkt 45 bis 50°/0,4 mbar (27 %, bezogen auf eingesetzte Methyldiacetoxybutenale).

## Vergleichsbeispiel 1

Ein Gemisch aus 2,9 g 2-Methyl-2,4-diacetoxy-3-butenal und 1,5 g 2-Methyl-4,4-diacetoxy-2-butenal wurde in 50 g Essigsäure gelöst, die 0,025 g konzentrierte Schwefelsäure enthielt und 20 Minuten bei 100 °C unter Stickstoff gerührt. Dann neutralisierte man die Schwefelsäure mit festem Natriumbicarbonat und zog die Essigsäure am Rotationsverdampfer ab. Durch Kugelrohrdestillation (70 bis 150 °C/1 mbar) des Rückstands wurden 1,1 g 3-Methyl-2,5-dihydrofuran-2-on (51 %, bezogen auf eingesetzte Methyldiacetoxybutenale), aber kein 2-Methyl-4,4-diacetoxy-2-butenal erhalten.

Vergleichsbeispiel 2

Ein Gemisch aus 2,8 g 2-Methyl-2,4-diacetoxy-3-butenal und 1,5 g 2-Methyl-4,4-diacetoxy-2-butenal wurde in 50 g Eisessig gelöst und in Gegenwart von 0,05 g p-Toluolsulfonsäure 4 Stunden auf 100 °C erhitzt. Gaschromatographisch war in dem Reaktionsgemisch als Hauptprodukt 3-Methyl-2,5-dihydrofuran-2-on, aber kein 2-Methyl-4,4-diacetoxy-2-butenal nachweisbar.

**Ansprüche**

1. Verfahren zur Herstellung von 2-Alkyl-4, 4-diacyloxy-2-butenalen der Formel

$$(R^2-\overset{O}{\overset{\|}{C}}-O)(R^3-\underset{O}{\underset{\|}{C}}-O)CH-CH=\overset{R^1}{\overset{|}{C}}-CH{=}O \qquad (I)$$

in der $R^1$ einen Alkylrest mit 1 bis 5 Kohlenstoffatomen und $R^2$ und $R^3$ ein Wasserstoffatom, einen aliphatischen Rest mit 1 bis 15 Kohlenstoffatomen, einen cycloaliphatischen Rest mit 5 bis 7 Kohlenstoffatomen oder einen aromatischen Rest bedeuten, dadurch gekennzeichnet, daß man 2-Alkyl-2,4-diacyloxy-3-butenale der Formel

$$R^2-\overset{O}{\overset{\|}{C}}-O-CH=CH-\overset{R^1}{\overset{|}{\underset{O-C(=O)-R^3}{\underset{|}{C}}}}-CH{=}O \qquad (II)$$

in der die Reste $R^1$ bis $R^3$ die obengenannte Bedeutung haben, in Abwesenheit von starken Säuren mit aliphatischen Carbonsäuren behandelt.

2. Verfahren nach Anspruch 1, dadurch gekennzeichnet, daß man als aliphatische Säuren Ameisensäure, Essigsäure, Propionsäure, Buttersäure, Valeriansäure oder Stearinsäure verwendet.

**Claims**

1. A process for the preparation of a 2-alkyl-4,4-diacyloxybut-2-enal of the formula

$$(R^2-\overset{O}{\overset{\|}{C}}-O)(R^3-\underset{O}{\underset{\|}{C}}-O)CH-CH=\overset{R^1}{\overset{|}{C}}-CH{=}O \qquad (I)$$

where $R^1$ is alkyl of 1 to 5 carbon atoms and $R^2$ and $R^3$ are each hydrogen, an aliphatic radical of 1 to 15 carbon atoms, a cycloaliphatic radical of 5 to 7 carbon atoms or an aromatic radical, wherein a 2-alkyl-2,4-diacyloxybut-3-enal of the formula

$$R^2-\overset{O}{\overset{\|}{C}}-O-CH=CH-\overset{R^1}{\overset{|}{\underset{O-C(=O)-R^3}{\underset{|}{C}}}}-CH{=}O \qquad (II)$$

where $R^1$, $R^2$ and $R^3$ have the above meanings, is treated with an aliphatic carboxylic acid in the absence of strong acids.

2. A process as claimed in claim 1, wherein formic acid, acetic acid, propionic acid, butyric acid, valeric acid or stearic acid is used as the aliphatic acid.

## Revendications

1. Procédé de préparation de 2-alkyl-4,4-diacyloxy-2-buténals de formule

$$\begin{matrix} R^2-\overset{O}{\overset{\|}{C}}-O \\ R^3-\underset{O}{\underset{\|}{C}}-O \end{matrix} \Big> CH-CH=\overset{R^1}{\overset{|}{C}}-C\overset{\nearrow O}{\searrow H} \qquad (I)$$

dans laquelle $R^1$ représente un groupe alkyle en C1-C5 et $R^2$ et $R^3$ représentent un atome d'hydrogène, un reste aliphatique en C1-C15, un reste cycloaliphatique en C5-C7 ou un reste aromatique, caractérisé en ce que l'on traite par des acides carboxyliques aliphatiques des 2-alkyl-2,4-diacyloxy-3-buténals de formule

$$R^2-\overset{O}{\overset{\|}{C}}-O-CH=CH-\overset{R^1}{\underset{O-C(=O)-R^3}{C}}-C\overset{\nearrow O}{\searrow H} \qquad (II)$$

dans laquelle les symboles $R^1$ à $R^3$ ont les significations indiquées ci-dessus, en l'absence d'acides forts.

2. Procédé selon la revendication 1, caractérisé en ce que l'on utilise en tant qu'acides aliphatiques les acides formique, acétique, propionique, butyrique, valérique ou stéarique.